(19) Europäisches Patentamt
European Patent Office
Office européen des brevets

(11) **EP 2 130 479 B1**

(12) **EUROPEAN PATENT SPECIFICATION**

(45) Date of publication and mention
of the grant of the patent:
**10.04.2019 Bulletin 2019/15**

(51) Int Cl.:
***A61B 1/00*** *(2006.01)*   ***A61B 1/008*** *(2006.01)*

(21) Application number: **08711855.0**

(22) Date of filing: **22.02.2008**

(86) International application number:
**PCT/JP2008/053087**

(87) International publication number:
**WO 2008/120508 (09.10.2008 Gazette 2008/41)**

(54) **DEVICE FOR CONTROLLING POSITION OF TREATMENT INSTRUMENT FOR ENDOSCOPE**

VORRICHTUNG ZUR KONTROLLE DER POSITION EINES BEHANDLUNGSINSTRUMENTS FÜR EIN ENDOSKOP

DISPOSITIF POUR COMMANDER LA POSITION D'UN INSTRUMENT DE TRAITEMENT POUR UN ENDOSCOPE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.03.2007 JP 2007089715**

(43) Date of publication of application:
**09.12.2009 Bulletin 2009/50**

(73) Proprietor: **Olympus Corporation
Tokyo 192-8507 (JP)**

(72) Inventor: **HASEGAWA, Jun
2-3 Kuboyama-cho, Hachioji-shi
Tokyo 192-8512 (JP)**

(74) Representative: **von Hellfeld, Axel
Wuesthoff & Wuesthoff
Patentanwälte PartG mbB
Schweigerstrasse 2
81541 München (DE)**

(56) References cited:
**WO-A2-2007/005367    JP-A- 2004 141 486
US-A- 5 432 543    US-A1- 2004 138 525
US-A1- 2005 228 221**

## Description

Technical Field

The present invention relates to a surgical instrument position control apparatus that controls the position of a surgical instrument inserted through a forceps channel in an endoscope.

Background Art

An endoscope is commonly known as an instrument for observing a possible lesion or the like in the body cavity. The endoscope includes an image pickup section at the distal end portion of an insertion section of the endoscope which is inserted into the body cavity, or in the main body of the apparatus. The endoscope thus displays a desired observation target on a monitor as an image. The insertion section is flexible and includes a channel (forceps channel) penetrating the endoscope from a proximal end side to the distal end. A surgical instrument such as a pair of forceps or an electric scalpel is inserted through an insertion port of the forceps channel as required. Thus, a surgeon can perform various surgical procedures on the lesion or the like while observing the endoscopic image.

Conventionally, the surgeon holds a manipulation section with one hand to manually manipulate the manipulation section to appropriately bend a bending section of the insertion section while observing the lesion or the like. The surgeon simultaneously manipulates the surgical instrument with the other hand. In recent years, to reduce the burden of endoscope manipulation on the surgeon, electrically-operated endoscopes have been proposed as disclosed in, for example, Japanese Patent No. 3007715. Furthermore, for surgical instruments, in order to not only reduce the burden of manipulation on the surgeon but also reduce operation time and thus burdens on a patient, electrically-operated robotized surgical instruments have been proposed as disclosed in, for example, Jpn. Pat. Appln. KOKAI Publication No. 2003-127076.

WO 2007/005367 A2 discloses several embodiments of telesurgical devices.

US 2004/138525 discloses an endoluminal tool deployment system.

US 2005/228221 discloses an endoscope information processor.

US 5,432,543 discloses an endoscopic image processing device for estimating three-dimensional shape of an object based on detection of same point on a plurality of different images.

Disclosure of Invention

As described above, the surgical instrument is normally inserted from the insertion port of the forceps channel to a forceps port at the distal end. The pair of forceps or electric scalpel projects from the distal end of the surgical instrument. The surgical instrument can be moved forward and backward through the forceps channel and moves integrally with the distal end portion of the endoscope. That is, as the distal end portion of the endoscope moves, the surgical instrument equivalently moves.

Namely, during operation, when the surgeon changes an observation visual field or the endoscope is moved by biological motion (peristaltic motion, a breath, the heart, or the like) in the body cavity of patient, the surgical instrument inserted from the proximal end side to distal end of the endoscope is integrally moved.

The surgeon thus needs to perform an operation of returning the distal end of the surgical instrument to the original position of the surgical procedure. The present invention is defined by the subject-matter of claim 1.

A surgical instrument position control apparatus for an endoscope comprises an endoscope allowing a biomedical tissue in the body cavity to be observed, a surgical instrument having a surgical section which is movable forward and backward through an insertion section of the endoscope and which allows the biomedical tissue to be operated on, and a movable section enabling the surgical section to be bent and moved forward and backward, a driving mechanism bending and moving forward and backward the movable section of the surgical section to move a position of the surgical section, a magnetic field generation section provided in the insertion section of the endoscope, an estimation section detecting a magnetic field generated by the magnetic field generation section, determining a position of the surgical section from a position of the magnetic field generation section based on the detected magnetic field, and estimating a moving direction and a moving amount of the surgical section in conjunction with movement of a distal end of the insertion section, and a control section drivingly controlling the driving mechanism based on the moving direction and amount of the surgical section so that the surgical section is returned to a position taken by the surgical section before the endoscope is moved.

Brief Description of Drawings

FIG. 1 is a diagram showing the general configuration of an endoscopic system in which a surgical instrument position control apparatus according to a first embodiment is mounted;

FIG. 2 is a flowchart illustrating a moving process according to the first embodiment;

FIG. 3A is a diagram illustrating estimation of motion of the distal end of the endoscope;

FIG. 3B is a diagram showing the external configuration of the distal end of the endoscope;

FIG. 3C is a diagram showing the position of a surgical section at the distal end of the endoscope during movement of the surgical section;

FIG. 4 is a diagram showing the general configuration of an endoscopic system in which a surgical instrument position control apparatus according to a second embodiment is mounted;

FIG. 5A is a diagram illustrating estimation of movement of the surgical section based on the estimated motion of the distal end of the endoscope in an endoscopic system according to a third embodiment;

FIG. 5B is a diagram showing the surgical procedure position of the endoscope in the endoscopic system according to the third embodiment;

FIG. 5C is a diagram showing the surgical procedure position of the endoscope observed after movement of the endoscope, in the endoscopic system according to the third embodiment;

FIG. 6 is a diagram showing the general configuration of an endoscopic system in which a surgical instrument position control apparatus according to a fourth embodiment is mounted; and

FIG. 7 is a flowchart illustrating an endoscopic system moving process according to a fifth embodiment.

Best Mode for Carrying Out the Invention Embodiments of the present invention will be described below with reference to the drawings.

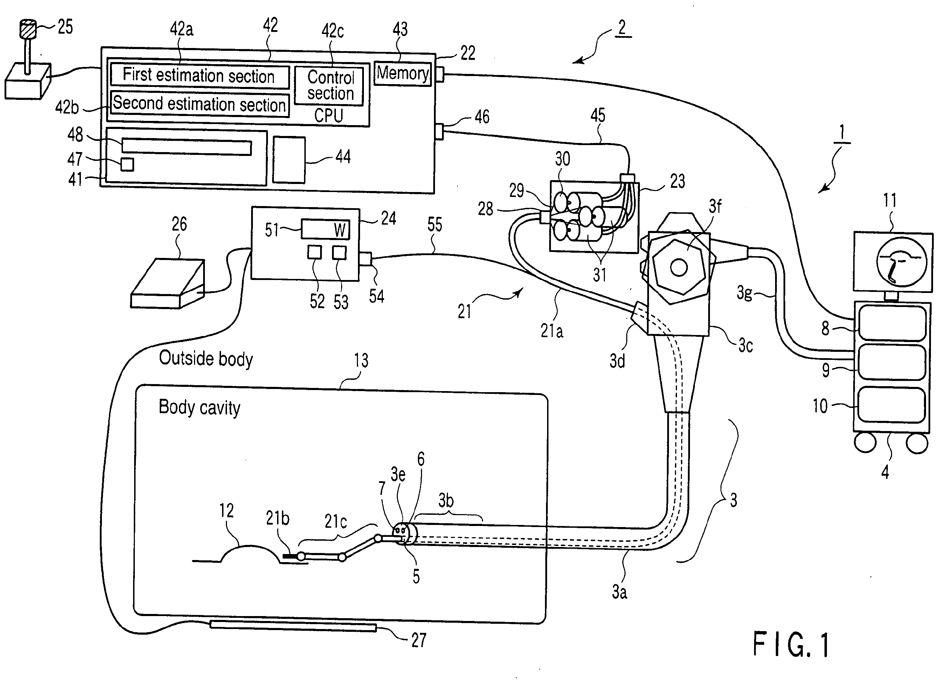

FIG. 1 is a diagram showing the general configuration of an endoscopic system in which a surgical instrument position control apparatus according to a first embodiment is mounted.

The endoscopic system is roughly composed of an endoscope 1 and a surgical instrument position control apparatus 2. The present invention is applicable to an electronic endoscope including an image pickup element at the distal end of an insertion section and an endoscope that takes an optical image guided through a fiber scope. In the embodiments described below, an electronic endoscope will be described by way of example.

An endoscope 1 is composed of an endoscope main body 3 and an apparatus main body 4. The endoscope main body 3 is composed of an insertion section 3a inserted into the body cavity, and a manipulation section 3c used to bend a bending section 3b provided on the distal end side of the insertion section 3a. The insertion section 3a includes, for example, a hole penetrating the insertion section 3a from an insertion port 3d that is open on the proximal end side, to a distal end 3e, that is, what is called a forceps port 5 for a forceps channel, and a light guide fiber 6 through which illumination light illuminating an observation visual field is propagated. Moreover, the distal end 3e has an image pickup section 7 including an image pickup element such as a CCD and an optical system. Image data on a lesion or the like obtained by the image pickup section 7 is transmitted, through the manipulation section 3c, to an image processing section 8 connected to the image pickup section 7, described below, of the apparatus main body 4 via a cable (the light guide fiber, an image signal line and a control signal line, and the like) 3g. The manipulation section 3c includes a manipulation dial 3f manipulated by the surgeon to bend the bending section 3b to bring a desired observation target (a lesion) 12 into an observation visual field (or an image pickup visual field).

The apparatus main body 4 is composed of an image processing section 8 executing various types of image processing and data processing on image data obtained by the image pickup section 7, a light source section 9 generating illumination light with which the observation visual field is irradiated through an illumination light window 6 via a light guide fiber, a control section 10 controlling the whole endoscopic system and executing arithmetic processing and the like, and a monitor 11 displaying picked-up images, data on the images, the status of the apparatus, operational instructions, and the like.

The surgical instrument position control apparatus 2 is composed of a surgical instrument 21 to which, for example, a high-frequency electric scalpel used to operate on the lesion 12 is attached, a surgical instrument control section 22 controlling the surgical instrument 21, a motor unit 23 driving the surgical instrument 21 based on a control signal from the surgical instrument control section 22, and a power source device 24 supplying high-frequency power to the high-frequency electric scalpel, which is the surgical instrument 21. Moreover, the surgical instrument position control apparatus 2 includes a joystick 25 connected to the surgical instrument control section 22 and serving as an input device manually operated by the surgeon to specify the position and posture of the surgical instrument 21, a foot switch 26 connected to the power source device 24 and operated by the surgeon's foot to specify that high-frequency power be supplied to the high-frequency electric scalpel, and a counter electrode plate 27 connected to the power source device 24 and stuck to the body surface of a patient 13.

The surgical instrument position control apparatus 2 is a master slave-type electric surgical member enabling the surgical section to be moved to a desired position in accordance with manipulation of the joystick 25. If with the surgical instrument 21 according to the present embodiment set to be positionally controlled, the surgeon or the like manipulates the joystick 25, the instruction for the manipulation of the joystick 25 is given top priority.

The surgical instrument 21 is composed of a surgical instrument insertion section 21a inserted through the forceps channel and which is movable forward and backward, the surgical instrument insertion section 21a having a flexible property, a surgical section 21b, for example, the high-frequency electric scalpel, which is used to operate on the lesion 12, and a movable section (surgical arm section) 21c coupled to the surgical instrument insertion section 21a and the surgical section 21b and enabling the surgical section 21b to be three-dimensionally moved. The surgical arm section 21c is configured to three-dimensionally move the surgical section 21b. In the present embodiment, the surgical arm section 21c adopts, for example, an articulated structure (bending, axial rotation, and the like) having a combination of a plurality of joints and short rods. Any of various components, for example, piezoelectric elements shaped like cylinders, can be used as the movable section.

The proximal end side of the surgical instrument insertion section 21a is connected to a motor unit 23 via a connection section 28. The motor unit 23 is composed of wires 29 one end of which is connected to the joints of the surgical arm section 21c through the surgical instrument insertion section 21a, pulleys 30 coupled to the other ends of the respective wires 29, and motors 31 each including a rotating shaft on which the corresponding one of the pulleys 30 is fittingly installed. The motors 31 are individually drivingly controlled by the surgical instrument control section 22. In this configuration, the surgical instrument control section 22 performs driving control to rotate the motors 31 so that the joints of the surgical arm section 21c are bent by the traction force of the wires 29 wound around the pulleys 30. Furthermore, an actuator (not shown in the drawings) provided in the motor unit 23 and made up of a motor or the like moves forward and backward and rotates the surgical instrument insertion section 21 so as to rotate and move forward and backward the surgical section 21b.

The surgical instrument control section 22 is composed of a function control input section 41 to which instructions given using the joystick 25 and conditions and parameters for function control are input, a central processing unit (CPU) 42, a memory 43 to which images, communication data, and the like are saved, a motor driver 44 drivingly controlling the motors 31 in the motor unit 23, and a motor unit control section 46 connected to the motor unit 23 via a cable 45 for communication.

CPU 42 is roughly separated into a first estimation section 42a executing arithmetic processing of estimating the amount of motion (motion vector) of the endoscope based on image data obtained by the image pickup section 7 of the endoscope, a second estimation section 42b which, from the motion and the motion amount, calculates a movement direction and a movement amount to be provided to the surgical arm section 21c in order to allow the surgical section 21b to return to a surgical procedure position in the endoscope taken by the surgical section 21b before the movement, and a control section 42c controlling the components of the surgical instrument position control apparatus 2. The memory 43 stores images received by an image receiving section receiving images taken by the image pickup section 7 located at the distal end of the endoscope, the results of calculations by CPU 42, communication data, and the like. Moreover, the function control input section 41 includes a switch 47 used to turn on and off the function of fixing the distal end of the surgical section 21b at a particular position of an observation target, and a display 48 showing the status of the function.

According to the amount by which the surgeon has manipulated the joystick 25, the surgical instrument control section 22 transmits a control signal allowing the respective motors 31 to be driven, to the motor driver 44, to rotate the motors 31. An encoder (not shown in the drawings) is attached to each of the motors 31 to measure the rotational speed of the motor. The encoder generates and transmits a signal corresponding to the rotational speed, to the surgical instrument control section 22. Thus, the encoder performs feedback control on the motor 31.

The power source device 24 includes a display 51 displaying the status of power supply, an output wattage input panel 52, an output mode selection panel 53, and a power output terminal 54. The power output terminal 54 supplies high-frequency power output by a power source unit (not shown in the drawings) provided in the power source device 24, to a high-frequency electric scalpel through a cable 55. The cable 55 is inserted through the surgical instrument insertion section 21a together with the above-described wires 29 and connected to the high-frequency electric scalpel.

The endoscopic system in which the surgical instrument position control apparatus configured as described above is mounted estimates the motion of the bending section 3b of the endoscope with respect to the lesion 12, that is, the observation target, based on time-sequentially adjacent images. Based on the estimated motion, the endoscopic system determines the motion of the surgical section 21b located at the distal end of the surgical instrument. Based on the motion of the surgical section 21b, the system calculates the amounts of operations of the joints such as bending, rotation, and forward and backward movements which are required to return the surgical section 21b to the position taken by the surgical section 21b before the movement. The system thus moves the surgical section 21b to the position taken by the surgical section 21b before the movement. Every time the surgical section 21b moves, the movement process is repeatedly executed. In the embodiments of the present invention, the position taken by the surgical section 21b before the movement is not a single point expressed in three-dimensional coordinates but a general position determined with the vicinity of the point taken into account.

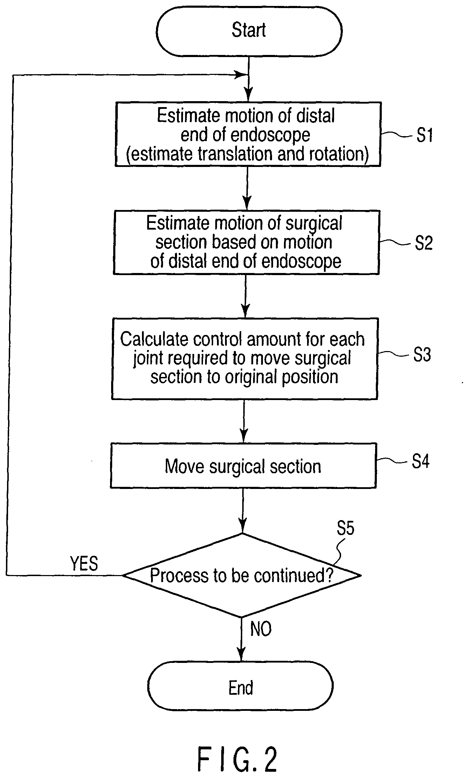

This moving process will be described in detail with reference to the flowchart shown in FIG. 2.

First, the surgical instrument control section 22 estimates the motion of the endoscope with respect to the target site based on time-sequentially adjacent image data obtained by the image pickup section 7 (step S1). Specifically, based on image data time-sequentially consecutively input by the endoscope, the surgical instrument control section 22 creates inter-image shift maps. In this technique, for example, shift maps are created for an optionally specified target site (for example, the lesion 12) in each screen obtained so that one inter-image shift map corresponds to estimation of one inter-image motion vector (a translation vector (h) and rotation matrix R: the translation vector is a unit vector). The motion vector, that is, the motion of the distal end of the endoscope, is estimated based on the shift map corresponding to each image. The translation vector and the rotation matrix are disclosed in, for example, Japanese Patent No. 3347385. Furthermore, a method of estimating the magnitude of a translation vector (H=kh) is disclosed in, for example, Jpn. Pat. Appln. KOKAI Publication No. 9-026547. The method is thus well known and will not be described in detail.

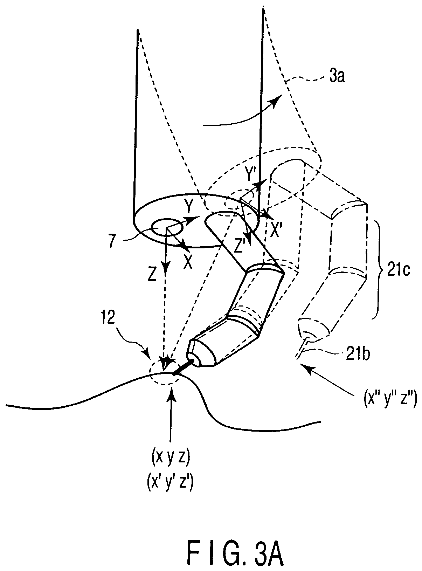

Then, the second estimation section of CPU 42 estimates the motion of the surgical section 21b based on the motion (the translation vector H and the rotation R) of the distal end of the endoscope (step S2). This motion will be described with reference to FIGS. 3A to (c). For the coordinates shown below, the origin (0,0,0) defines an X-axis, a Y-axis, and a Z-axis around the position of a view point set by the image pickup section 7 and optical system provided at the distal end of the endoscope.

As shown in FIG. 3A, it is assumed that for example, the surgical section 21b is located at the position (x,y,z) of the lesion 12 before the endoscope is moved for a certain reason. Then, the position (x',y',z') taken by the surgical section 21b before the movement is expressed as follows:

$$\begin{pmatrix} x' \\ y' \\ z' \end{pmatrix} = R^{-1} \begin{pmatrix} x - H_x \\ y - H_y \\ z - H_z \end{pmatrix}$$

wherein H: translation of the endoscope and R: rotation of the endoscope. In contrast, the position (x",y",z") taken by the surgical section 21b after the movement is expressed as follows.

$$\begin{pmatrix} x'' \\ y'' \\ z'' \end{pmatrix} = \begin{pmatrix} x \\ y \\ z \end{pmatrix}$$

At this time, the positional relationship between the distal end of the endoscope and the surgical arm section 21c (the portion extending from the forceps port 5 of the forceps channel) remains unchanged. Thus, the surgical section 21b has substantially moved from the position (x',y'z') to the position (x",y",z").

The position (x,y,z) of the surgical section 21b is determined from the angle of each of the joints of the surgical arm section 21c determined from the rotational amount of the motor 31 moving the joint (the rotational amount is calculated from a value from the encoder connected to each of the motors 31), as well as the length of the joint and the connection status of the joint. However, the system needs to be initialized so that the motion of the surgical section 21b matches a coordinate system based on the distal end of the endoscope (for example, the system is initialized such that the UP/DOWN direction of bending of the endoscope matches the UP/DOWN direction of the surgical section 21b). Furthermore, the position (x',y',z') taken by the surgical section 21b after the endoscope has been moved is calculated using Equations (1) and (2). From the resulting position (x',y',z'), joint length, and joint connection status, the angle of each joint can be determined based on reverse kinematics.

The reverse kinematics is a method of estimating a specific value for each joint (the angle of the joint or the like) from information on the position and posture of a manipulator (surgical instrument) in a work space. A joint parameter Φ for joints 1, 2, ..., n is specified as follows.

$$\Phi = (\theta_1, \theta_2, \cdots, \theta_n)^T$$

The position and posture of the manipulator are specified as follows.

$$Ep = \left( x_{Ep}, y_{Ep}, z_{Ep}, Roll_{Ep}, Yaw_{Ep}, Pitch_{Ep} \right)^T$$

Then, the relationship between the joint parameter and the position and posture of the manipulator is expressed by:

$$E_p = A(\Phi)$$

Here, a target P for the position and posture of the manipulator is specified as follows.

$$Pp = \left(x_{Pp}, y_{Pp}, z_{Pp}, Roll_{Pp}, Yaw_{Pp}, Pitch_{Pp}\right)^T$$

Then, to set the manipulator in a condition indicated by $P_p$, $\Phi$ needs to be determined which satisfies:

$$P_p = A(\Phi).$$

However, these relations are nonlinear. Thus, in order to find the value of $\Phi$, a Jacobian determinant $J(\Phi)$ obtained by partially differentiating Ep by the elements of $\Phi$ is generally determined.

$$J(\Phi) = \begin{pmatrix} dx_{ep}/d\theta_1 & dx_{ep}/d\theta_2 & \cdots & dx_{ep}/d\theta_n \\ dy_{ep}/d\theta_1 & dy_{ep}/d\theta_2 & \cdots & dy_{ep}/d\theta_n \\ dz_{ep}/d\theta_1 & dz_{ep}/d\theta_2 & \cdots & dz_{ep}/d\theta_n \\ dRoll_{ep}/d\theta_1 & dRoll_{ep}/d\theta_2 & \cdots & dRoll_{ep}/d\theta_n \\ dYaw_{ep}/d\theta_1 & dYaw_{ep}/d\theta_2 & \cdots & dYaw_{ep}/d\theta_n \\ dPitch_{ep}/d\theta_1 & dPitch_{ep}/d\theta_2 & \cdots & dPitch_{ep}/d\theta_n \end{pmatrix}$$

Then, $\Phi$ satisfying $P_p = A(\Phi)$ is determined from Equation 9 by convergence calculation.

$$\dot{\Phi} = J(\Phi)^{-1} \dot{E}_p$$

from

$$P_p = A(\Phi)$$

Based on the calculated operation amount, the joints of the surgical arm section 21c are bent to move the surgical section 21b to the position of the lesion 12 (step S4).

The surgical arm section 21c is bent, rotated, and moved forward and backward so as to hold the position of the surgical section 21b. This is repeated until the surgical section 21b completes the surgery on the lesion 12 (step S5).

That is, an inverse problem is used to determine the required bending, rotation, or forward or backward movement so that the required movement can be performed.

As described above, according to the present embodiment, even if during surgery with the surgical instrument, the surgeon moves the observation visual field for the surgeon's own convenience or biological motion in the body cavity moves the endoscope, the surgical section of the surgical instrument is prevented from being moved away from the target site being operated on. The position of the surgical section thus remains unchanged. This eliminates the need for the surgeon's moving operation of returning the surgical section to the original position. Thus, the surgeon can concentrate on the surgical procedure and have the burden of the manipulation and fatigue reduced.

Now, a second embodiment of the present invention will be described.

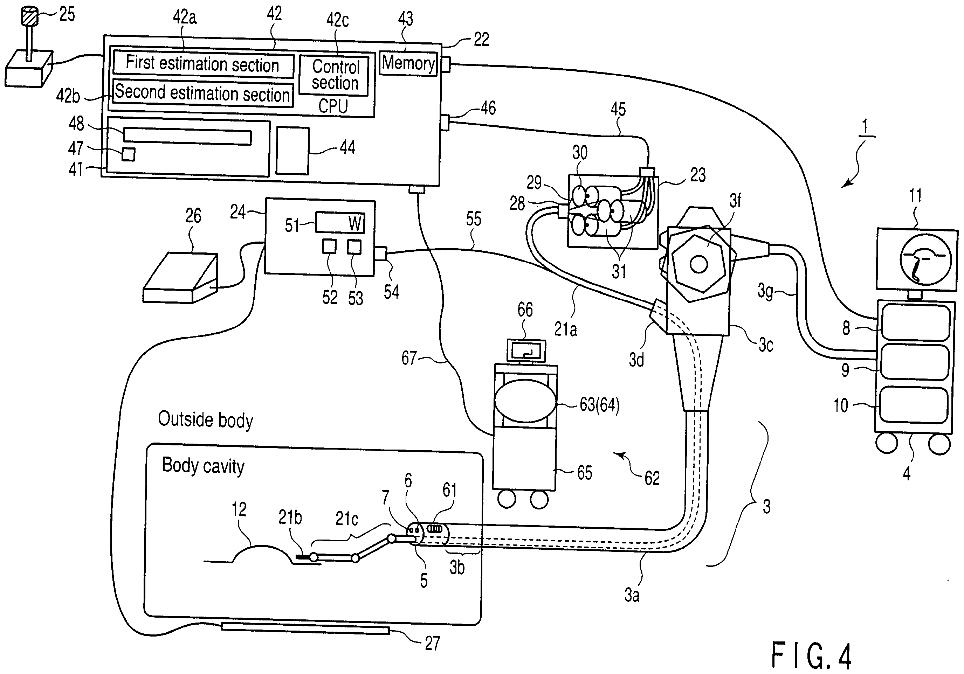

FIG. 4 is a diagram showing the general configuration of an endoscopic system in which a surgical instrument position control apparatus according to the present embodiment is mounted.

The endoscopic system corresponds to the configuration of the endoscopic system according to the first embodiment shown in FIG. 1 described above and in which a magnetic field generation coil is provided on the distal end side of the endoscope main body 3. In the present endoscopic system, a magnetic field generated by the magnetic field generation coil is detected by an endoscope shape observation device to estimate the position of the distal end of the endoscope. In the present embodiment, the components other than the magnetic field generation coil and the endoscope shape observation device are equivalent to the corresponding ones in the endoscopic system shown in FIG. 1. Thus, these components are denoted by the same reference numerals and will not be described below.

As shown in FIG. 4, the endoscope main body 3 includes at least one magnetic field generation coil 61 at a position closer to the distal end of the insertion section 3a than the bending section 3b. Furthermore, an endoscope shape observation device 62 is located near the patient. The endoscope shape observation device 62 is composed of a magnetic field detection unit 63, a position estimation section 65, and a monitor 66.

The magnetic field detection unit 63 includes a plurality of magnetic field detection coils 64 (64a, 64b, ..., 64n) arranged inside a housing to detect a magnetic field generated by the magnetic field generation coil 61. The position estimation section 65 receives data on the magnetic field detected by the magnetic field detection coil 64 to estimate the position of the magnetic field generation coil 61. The estimated position of the magnetic field generation coil 61 is a value in a coordinate system based on the magnetic field detection unit 63. This is disclosed in Japanese Patent No. 3571675 in detail. Estimated positional information on the magnetic field generation coil 61 is transmitted to the surgical instrument control section 22 through a signal line 67. CPU 42 in the surgical instrument control section 22 calculates the position of the distal end of the insertion section 3a (the view point position set based on the image pickup element and the optical system) the image of which is taken by the image pickup section 7, based on the positional relationship (design value) with the magnetic field generation coil 61. CPU 42 then calculates the amount of movement (the magnitude of the corresponding translation vector) between images based on the calculated position of the distal end of the insertion section 3a. CPU 42 then allows the first estimation section 42a to estimate the moving direction and amount of the insertion section 3a of the endoscope using the calculated moving amount (the magnitude of the translation vector).

In the present configuration example, the magnetic field detection coil 64 is provided inside the housing of the magnetic field detection unit 63. However, the magnetic field detection coil 64 may be located near and around the patient.

According to the endoscopic system in which the surgical instrument position control apparatus according to the second embodiment described above is mounted, the motion amount (the magnitude of the translation vector) of the endoscope can be accurately calculated. Thus, the surgical section 2c can be accurately moved.

Now, a third embodiment of the present invention will be described.

In the third embodiment, the movement of the distal end of the endoscope is estimated based on the matching between images to determine the position of the surgical instrument. Then, the surgical instrument is moved parallel to the image pickup surface of the image pickup element back to the original position. The configuration of the present embodiment is the same as that of the first embodiment as shown in FIG. 1.

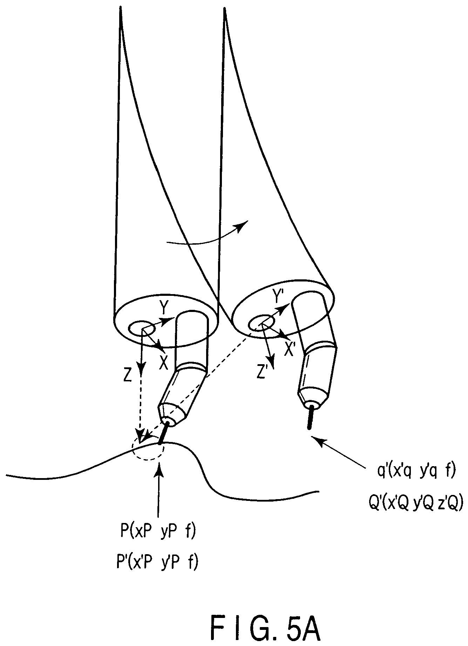

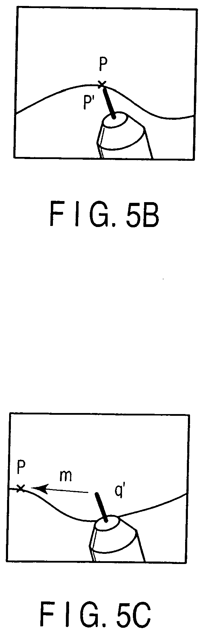

As shown in FIGS. 5A, 5B, and 5C, $p(x_p, y_p, f)$ [f: the focal distance of the optical system in the image pickup section 7] denotes the position of the distal end (the surgical section 21b or lesion 12) of the surgical instrument 21 in the observation visual field, on the screen obtained before movement, of the endoscope being used to operate on the lesion. $P'(x_p', y_p', z_p')$ denotes the position of the surgical procedure after the endoscope has been moved (the position taken by the surgical instrument 21 before the endoscope has been moved). In an image taken after the endoscope has been moved, the position (p) taken by the surgical section 21 before the movement can be determined based on the matching between images. The matching is a process of using, as a template, a part of an image taken before the movement which corresponds to the vicinity of the position (p) where the distal end of the surgical section 21 is present, to search an image taken after the movement of the endoscope, thus determining the position. If the surgical instrument 21 is present in the template, the region of the surgical section 21 is detected and excluded from a target for a process of calculating the level of the matching between the images. The movement, on the screen, from the position $q'(x_q', y_q', f)$ taken by the distal end of the surgical section 21 after the movement of the endoscope to the position $p'(x_p', y_p', f)$ of the surgical procedure observed after the movement of the endoscope is as shown in:

$$m = \begin{pmatrix} x'_q - x'_p \\ y'_q - y'_p \\ 0 \end{pmatrix}$$

where f denotes the focal distance of the image pickup section 7 incorporated into the tip of the endoscope.

For the three-dimensional position Q'(XQ',YQ'ZQ') of the distal end of the surgical section 21, the magnitudes of the bending, rotation, and forward and backward movements of each of the joints of the surgical section 21 are determined from values from the encoder connected to the respective motors. If the value ZQ' of the Z component of the distal end of the surgical section 21 obtained after the movement of the endoscope is used to move the distal end of the surgical section 21 parallel to the image pickup surface of the image pickup element of the image pickup section 7 to the vicinity of the position of the surgical procedure (the position of the lesion 12: the position where the distal end of the surgical section 21 is present before the movement of the endoscope), the direction and magnitude of the movement are expressed as shown in:

$$M \;=\; \frac{z'_q}{f} \begin{pmatrix} x'_q - x'_p \\ y'_q - y'_p \\ f \end{pmatrix} \;.$$

As described above, according to the endoscopic system in which the surgical instrument position control apparatus according to the present embodiment is mounted, if the endoscope is moved parallel to the image pickup surface of the image pickup element, the motion of the endoscope can be estimated more quickly than in the first and second embodiments. Thus, the surgical section can be moved at a speed close to that in real time.

Now, a fourth embodiment of the present invention will be described.

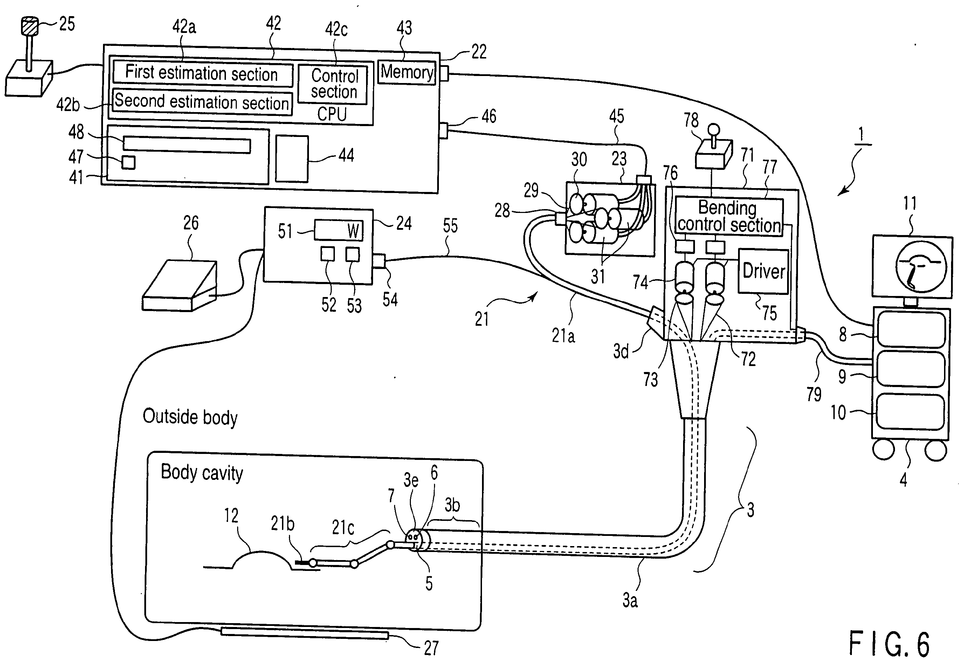

The endoscope according to the fourth embodiment of the present invention shown in FIG. 6 is configured such that the bending section of the endoscope main body is electrically bent. In the present embodiment, the endoscope main body is electrically driven, and the other components are equivalent to the corresponding ones in the first embodiment shown in FIG. 1 described above. Thus, these components are denoted by the same reference numerals and will not be described below.

An electrically-operated bending manipulation section 71 in the endoscope main body 3 includes a plurality of wires 72 one end of each of which is connected to the bending section 3b, a plurality of pulleys 73 each coupled to the other end of the corresponding wire 72, motors 74 each including a rotating shaft on which the corresponding pulley 73 is fittingly installed, a driver 75 individually driving the respective motors 74, encoders 76 each provided in the corresponding motor, and a bending control section 77 controlling the motor driver 75 based on values detected by the encoders 76. Moreover, the bending control section 77 is connected to a bending joystick 78 used to specify a bending operation.

Furthermore, the electrically-operated bending manipulation section 71 is connected to the apparatus main body 4 via a cable 79. The cable 79 includes a light guide fiber through which illumination light is transmitted, and signal lines including an image signal line and a control signal line. Furthermore, in the configuration example described in the present embodiment, the joystick is provided in each of the endoscope and the surgical instrument. However, these manipulation functions are collectively provided in one joystick. Moreover, the present embodiment may include the magnetic field generation coil and endoscope shape observation device provided in the endoscopic system according to the second embodiment.

The amounts by which the endoscope main body and the surgical instrument are bent are determined from the amounts (encoder output values) by which the motors pulling the respective wires are rotated. Thus, the motion of the distal end of the endoscope is estimated from the rotational amounts of the endoscope motors. The distal end of the surgical instrument is moved based on the estimated values.

According to the endoscopic system in which the surgical instrument position control apparatus according to the present embodiment is mounted as described above, not only the effects of the first and third embodiments are exerted but also the bending operations of the surgical instrument and the endoscope are specified using the manipulation switch made up of a joystick or the like and which can be easily manipulated. This enables a reduction in the surgeon's fatigue and burden. Moreover, when the present embodiment includes the magnetic field generation coil and endoscope shape observation device in the endoscopic system according to the second embodiment, the position detection based on image processing is avoided, eliminating the need for advanced calculations by CPU. Thus, the motion of the distal end of the endoscope can be easily estimated, enabling the distal end (surgical section) of the surgical instrument to be moved at a speed close that in real time. Furthermore, the motion amount (the magnitude of the translation vector) of the endoscope can be accurately calculated, allowing the surgical section 2c to be accurately moved.

In the configuration example in the present embodiment, the electrically-operated endoscope main body is used. However, even a non-electrically-operated, normal endoscope main body may be used to exert similar effects by providing a sensor at the insertion port 3d of the forceps channel or the forceps port 5 to detect the traction amount of the wire, and providing a mechanism detecting the rotational amount of each manipulation dial 3f installed in the manipulation section to determine the traction amount of the wire.

Now, a fifth embodiment of the present invention will be described.

In an endoscopic system in which a surgical instrument position control apparatus according to the present embodiment is mounted, if the movement amount of the endoscope is smaller then a preset threshold value, the position of the surgical instrument is maintained without change. This is to prevent a possible situation where if the distal end of the surgical section is always moved in conjunction with movement of the endoscope, the distal end of the surgical instrument is always vibrated by noise, calculation errors, or the like, making the surgical procedure difficult. Software (program) can be configured to provide a threshold for the movement amount of the endoscope according to the present embodiment. Thus, the above-described arrangement is applicable to the configuration of any of the above-described first to fourth embodiments.

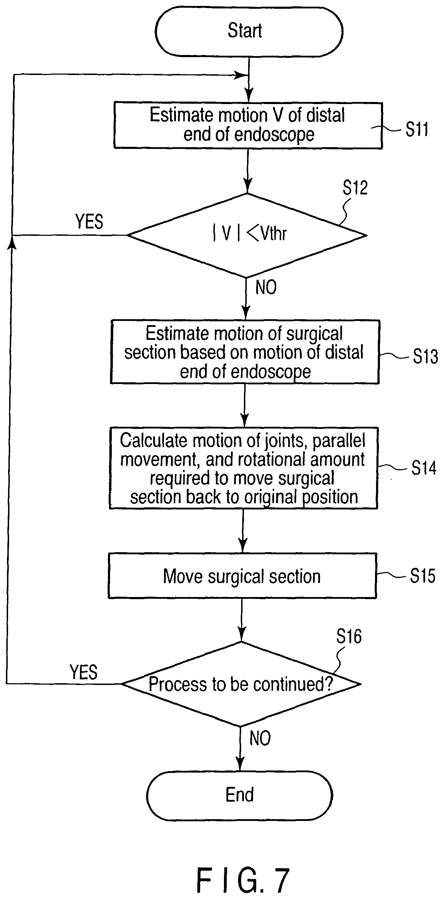

This moving process will be described in detail with reference to the flowchart shown in FIG. 7. In this case, an example in which the moving process is applied to the endoscopic system according to the first embodiment will be described.

First, the motion vector of the endoscope main body 3 with respect to the target site is estimated based on time-sequentially adjacent images obtained (step S11). Specifically, inter-image shift maps are created based on time-sequentially consecutively input image data obtained by the image pickup section 7. For an optionally specified target site (for example, the lesion 12) in the screens, one inter-image shift map corresponds to estimation of one inter-image motion vector (a translation vector (h) and rotation matrix R: the translation vector is a unit vector). The motion vector, that is, the motion (relative motion) of the distal end of the endoscope, is estimated based on the shift map corresponding to each image. The magnitude of the translation vector is then determined to calculate the absolute motion V of the distal end of the endoscope.

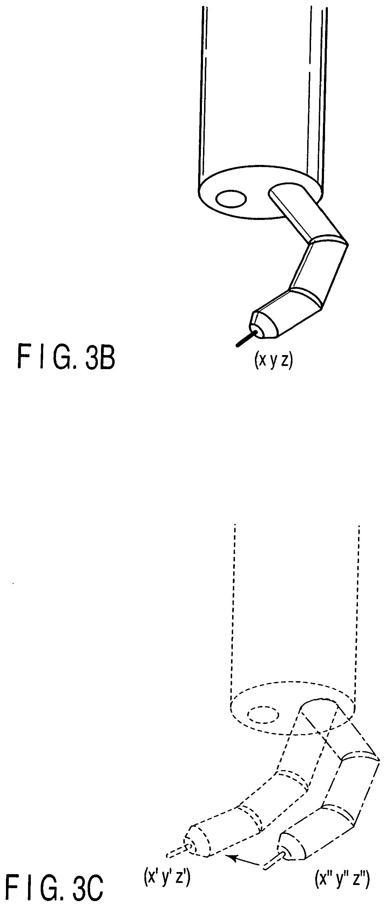

The calculated absolute value $|V|$ of the motion of the distal end of the endoscope is compared with an empirically or experimentally determined threshold Vthr (step S12). In the comparison, if the motion $|V|$ of the distal end of the endoscope is smaller than the threshold Vthr (YES), the device determines that this level of movement of the endoscope does not cause the process of moving the surgical instrument to be executed. The device returns to step S11 to continuously estimate the motion of the distal end of the endoscope. On the other hand, if the motion $|V|$ is larger than the threshold Vthr (NO), the device determines that the process of moving the surgical instrument is to be executed. The device shifts to step S13. Step S13 corresponds to step S2 in FIG. 2 described in the first embodiment. The motion of the surgical section 21b is estimated based on the motion (the translation vector H and the rotation R: see FIG. 3) of the distal end of the endoscope (step S13). As shown in FIG. 3, the device determines, for example, the position (x,y,z) of the surgical section 21b to be moved by the endoscope and the position (x',y'z') taken by the surgical section 21b after the movement of the endoscope and before the surgical section 21b is positionally corrected. In connection with this, the position (x",y",z") of the moved surgical section 21b is determined. Then, based on the calculated positions of the surgical section 21b, the device calculates the amount of movement from the coordinate position (x",y",z") taken by the surgical section 21b after the movement of the endoscope to the position (x',y',z'), that is, the amounts of operations, such as bending, rotation, and forward and backward movements, of the joints of the surgical arm section 21c which amounts are required to move the surgical section 21b to the original position of the lesion 12 (step S14).

Then, based on the calculated operation amounts, the joints of the surgical arm section 21c are bent to move the surgical section 21b to the position of the lesion 12 (step S15). The surgical arm section 21c is bent, rotated, and moved forward and backward so as to hold the position of the surgical section 21b. This is repeated until the surgical section 21b completes the surgery on the lesion 12 (step S16).

As described above, according to the present embodiment, the particular threshold is provided for the motion of the endoscope. This prevents a possible situation where the distal end of the surgical instrument is always vibrated by noise, calculation errors, or the like, making the surgical procedure difficult. Thus, when the surgeon operates on the patient, possible unwanted vibration is prevented. Consequently, the surgeon can concentrate on the surgical procedure and have the burden of the manipulation and fatigue reduced.

Even if the surgeon moves the observation visual field for the surgeon' own convenience or biological motion in the body cavity moves the endoscope, the surgical section of the surgical instrument is prevented from being moved away from the target site being operated on. The position of the surgical section thus remains unchanged.

The present invention can provide the surgical procedure position control apparatus for the endoscope which, even when the distal end of the endoscope is moved during surgery with the surgical instrument, allows the surgical instrument to be prevented from being moved away from the site being operated on, enabling the surgical procedure to be continued.

When the endoscope is moved in response to the surgeon's manipulation or biological motion in the body cavity, the surgical instrument position control apparatus estimates the motion of the distal end of the insertion section of the endoscope with respect to the target site. Based on the motion of the distal end of the insertion portion, the surgical instrument position control apparatus further estimates the motion of the surgical instrument. Moreover, the surgical instrument position control apparatus calculates the amounts of operations, such as bending, rotation, and forward and backward movements, of the joints located at the distal end of the surgical instrument which amounts are required to move the surgical section from the position taken by the surgical instrument after the movement of the endoscope to the surgical procedure position. The surgical instrument position control apparatus can thus operate the joints to move the distal end of the surgical instrument to the original position so as to hold the position of the surgical instrument every time the endoscope is moved. This enables a reduction in the burden of the manipulation and fatigue.

Furthermore, the surgical instrument position control apparatus includes the magnetic field generation coil provided at the distal end of the endoscope. The surgical instrument position control apparatus estimates the position and motion of the distal end of the endoscope based on a generated magnetic field, to estimate the motion of the surgical instrument based on the motion of the distal end. Thus, the surgical instrument position control apparatus can thus quickly calculate the operation amounts required to move to the original surgical procedure position and return the surgical instrument to the surgical procedure position. Moreover, the threshold for the detection of movement of the endoscope is provided. This prevents possible vibration associated with the unwanted position holding operation for the surgical instrument performed as a result of noise, calculation errors, or the like. Therefore, the surgical procedure is prevented from being made difficult.

**Claims**

1.  A surgical instrument position control apparatus (2) for an endoscope (1) comprising:

    an endoscope body (3) allowing a biomedical tissue in body cavity to be observed;
    a surgical instrument (21) having a surgical section (21b) which is movable integrally with movement of a distal end (3e) of the endoscope (1), and is movable forward and backward through a channel of an insertion section (3a) of the endoscope (1) and which allows the biomedical tissue to be operated on, and a movable section (21c) to bend and move forward and backward the surgical section (21b);
    a driving mechanism (23) adapted for bending and moving forward and backward the movable section (21c) of the surgical section (21b) to move a position of the surgical section (21b);
    a first estimation section (42a) adapted to execute arithmetic processing of estimating the motion of the distal end (3e) of the endoscope (1) based on image data obtained by the image pickup section (7) of the endoscope (1);
    a second estimation section (42b) adapted for estimating a moving direction and a moving amount of the surgical section (21b) based on the motion of the distal end (3e) of the endoscope (1) estimated by the first estimation section (42a); and
    a control section (42c) adapted for drivingly controlling the driving mechanism (23) based on the moving direction and moving amount of the surgical section (21b), which is based on the moving direction and moving amount of the surgical section (21b) estimated by the second estimation section (42b), so that the surgical section (21b) is returned to a position taken by the surgical section (21b) before the endoscope (1) is moved.

2.  The surgical instrument position control apparatus for the endoscope according to claim 1, wherein the movable section (21c) of the surgical section (21b) has an articulated structure including a plurality of joints and rods connecting the joints together.

3.  The surgical instrument position control apparatus for the endoscope according to claim 1, wherein:

    the first estimation section (42a) is adapted for estimating the motion of the endoscope body (3); and
    the second estimation section (42b) is adapted for estimating the moving direction and moving amount of the surgical section (21b) of the surgical instrument (21) with respect to the motion of the endoscope (1), based on a preset positional relationship between the endoscope body (3) and the surgical instrument (21),
    wherein a position of the surgical section (21b) is estimated based on the motion of the surgical section (21b) on an image picked up by an image pickup section (7) provided at a distal end (3e) of the insertion section (3a) of the endoscope (1), angle information on joints of the movable section (21c) of the surgical instrument (21) which information is loaded into the control section (42c), and a status of the surgical instrument (21) based on estimated translation movement information and rotational information.

4.  The surgical instrument position control apparatus for the endoscope according to claim 1, wherein the driving mechanism (23) comprises:

    a plurality of wires (29) one end of each of which is connected to a corresponding one of the joints of the movable section (21c); and
    an electrically-operated driving mechanism adapted to be controlled by the control section (42c) and connected to another end of each of the wires (29) to pull and loosen any of the wires (29) to bend the movable section (21c) in a desired direction,
wherein the control section (42c) is adapted, based on the moving direction and moving amount of the surgical section (21b) determined based on the position estimated by the estimation sections, to drive the electrically-operated driving mechanism to return the surgical instrument (21) to the position taken by the surgical section (21b) before the endoscope (1) is moved.

5. The surgical instrument position control apparatus for an endoscope according to claim 1, further comprising:

a magnetic field generation section (61) provided in the insertion section (3a) of the endoscope (1);
wherein the second estimation section is adapted for detecting a magnetic field generated by the magnetic field generation section (61), determining a position of the surgical section (21b) from a position of the magnetic field generation section (61) based on the detected magnetic field, and estimating a moving direction and a moving amount of the surgical section (21b) in conjunction with movement of the endoscope (1).

6. The surgical instrument position control apparatus for the endoscope according to claim 1, further configured such that, upon estimating that the movement of the endoscope (1) is parallel to an image pickup surface of an image pickup section (7) provided at a distal end side (3e) of the insertion section (3a), the estimation sections of the surgical instrument (21) fix a distance in a depth direction orthogonal to the image pickup surface, to a focal direction of an optical system in the image pickup section, to estimate the moving direction and moving amount of the surgical section (21b).

7. The surgical instrument position control apparatus for the endoscope according to claim 1, wherein the surgical instrument (21) is insertable through the insertion section (3a) of the endoscope (1) so as to be movable forward and backward, the insertion section (3a) having an electrically-operated driving mechanism (71) adapted for bending, in a desired direction, a bending section (3b) provided at a distal end of the insertion section (3a) of the endoscope (1).

8. The surgical instrument position control apparatus for the endoscope according to claim 1, wherein the estimation sections are adapted to hold a position of the surgical instrument (21) if the moving amount of the insertion section (3a) of the endoscope (1) is smaller than a preset threshold.

9. The surgical instrument position control apparatus for the endoscope according to claim 1, **characterized in that** for an optionally specified target site in a screen in images picked up by an image pickup section (7), the control section (22) is adapted to estimate motion of the distal end (3e) of the endoscope (1) between time-sequentially adjacent images to determine magnitude of a translation vector in the motion to calculate absolute motion of the distal end (3e) of the endoscope (1).

10. The surgical instrument position control apparatus for the endoscope according to claim 1, wherein the insertion section (3a) of the endoscope (1) has an electrically-operated driving mechanism comprising an articulated structure having a plurality of joints each of which is bent by pulling and loosening a wire coupled to a pulley attached to a shaft of a motor, the electrically-operated driving mechanism being adapted to be driven by the motor in accordance with an instruction from the control section (42c) to perform a moving operation and a posture holding operation.

11. The surgical instrument position control apparatus for the endoscope according to claim 4, **characterized in that** an electrically-operated driving mechanism (71) for the insertion section (3a) of the endoscope (1) and the electrically-operated driving mechanism for the movable section (21c) of the surgical section (21b) are master slave type devices each including an input device (25, 78) adapted to be manipulated by a surgeon to remotely specify positions and postures of the insertion section and the surgical section (21b).

12. The surgical instrument position control apparatus for the endoscope according to claim 4, wherein the estimation sections are configured to estimate the moving amount of the surgical section (21b) based on operation amounts of bending, rotation, and forward and backward movements of each of the joints.

**Patentansprüche**

1. Vorrichtung (2) zum Steuern der Position eines chirurgischen Instruments für ein Endoskop (1), umfassend:

einen Endoskopkörper (3), der ermöglicht, dass ein biomedizinisches Gewebe in einem Körperhohlraum beobachtet wird;

ein chirurgisches Instrument (21) mit einem chirurgischen Teilabschnitt (21b), der mit einer Bewegung eines distalen Endes (3e) des Endoskops (1) integral beweglich ist und der durch einen Kanal eines Einführteilabschnitts (3a) des Endoskops (1) vor- und rückwärts beweglich ist und der ermöglicht, dass das biomedizinische Gewebe bearbeitet wird, und mit einem beweglichen Teilabschnitt (21c), um den chirurgischen Teilabschnitt (21b) zu biegen und vorwärts und rückwärts zu bewegen;

einen Antriebsmechanismus (23), der so ausgelegt ist, dass er den beweglichen Teilabschnitt (21c) des chirurgischen Teilabschnitts (21b) biegt und vorwärts und rückwärts bewegt, um eine Position des chirurgischen Teilabschnitts (21b) zu bewegen;

einen ersten Schätzungsteilabschnitt (42a), der so ausgelegt ist, dass er eine arithmetische Verarbeitung des Schätzens der Bewegung des distalen Endes (3e) des Endoskops (1) auf Basis von Bilddaten durchführt, die vom Bildaufnahmeteilabschnitt (7) des Endoskops (1) erhalten werden;

einen zweiten Schätzungsteilabschnitt (42b), der so ausgelegt ist, dass er eine Bewegungsrichtung und einen Bewegungsbetrag des chirurgischen Teilabschnitts (21b) auf Basis der Bewegung des distalen Endes (3e) des Endoskops (1) schätzt, die vom ersten Schätzungsteilabschnitt (42a) geschätzt wird; und

einen Steuerteilabschnitt (42c), der so ausgelegt ist, dass er den Antriebsmechanismus (23) auf Basis der Bewegungsrichtung und des Bewegungsbetrags des chirurgischen Teilabschnitts (21b) antriebssteuert, was auf der Bewegungsrichtung und dem Bewegungsbetrag des chirurgischen Teilabschnitts (21b) basiert, die vom zweiten Schätzungsteilabschnitt (42b) geschätzt werden, so dass der chirurgische Teilabschnitt (21b) in eine Position zurückgebracht wird, die der chirurgische Teilabschnitt (21b) einnimmt, bevor das Endoskop (1) bewegt wird.

2. Vorrichtung zum Steuern der Position eines chirurgischen Instruments für das Endoskop nach Anspruch 1, wobei der bewegliche Teilabschnitt (21c) des chirurgischen Teilabschnitts (21b) eine Gelenkstruktur aufweist, die eine Mehrzahl von Gelenken und Stangen umfasst, die die Gelenke miteinander verbinden.

3. Vorrichtung zum Steuern der Position eines chirurgischen Instruments für das Endoskop nach Anspruch 1, wobei:

der erste Schätzungsteilabschnitt (42a) so ausgelegt ist, dass er die Bewegung des Endoskopkörpers (3) schätzt; und

der zweite Schätzungsteilabschnitt (42b) so ausgelegt ist, dass er die Bewegungsrichtung und den Bewegungsbetrag des chirurgischen Teilabschnitts (21b) des chirurgischen Instruments (21) in Bezug auf die Bewegung des Endoskops (1) auf Basis einer vorab eingestellten Positionsbeziehung zwischen dem Endoskopkörper (3) und dem chirurgischen Instrument (21) schätzt,

wobei eine Position des chirurgischen Teilabschnitts (21b) auf Basis der Bewegung des chirurgischen Teilabschnitts (21b) auf einem Bild, das von einem Bildaufnahmeteilabschnitt (7) aufgenommen wird, der an einem distalen Ende (3e) des Einführteilabschnitts (3a) des Endoskops (1) bereitgestellt ist, einer Winkelinformation zu Gelenken des beweglichen Teilabschnitts (21c) des chirurgischen Instruments (21), wobei die Information in den Steuerteilabschnitt (42c) geladen wird, und eines Status des chirurgischen Instruments (21) auf Basis einer geschätzten Translationsbewegungsinformation und Drehinformation geschätzt wird.

4. Vorrichtung zum Steuern der Position eines chirurgischen Instruments für das Endoskop nach Anspruch 1, wobei der Antriebsmechanismus (23) umfasst:

eine Mehrzahl von Kabeln (29), wobei ein Ende jedes dieser mit einem entsprechenden der Gelenke des beweglichen Teilabschnitts (21c) verbunden ist; und

einen elektrisch betriebenen Antriebsmechanismus, der so ausgelegt ist, dass er vom Steuerteilabschnitt (42c) gesteuert wird, und der mit einem anderen Ende jedes der Kabel (29) verbunden ist, um an einem beliebigen der Kabel (29) zu ziehen und dieses zu lockern, so dass der bewegliche Teilabschnitt (21c) in eine gewünschte Richtung gebogen wird,

wobei der Steuerteilabschnitt (42c) so ausgelegt ist, dass er den elektrisch betriebenen Antriebsmechanismus auf Basis der Bewegungsrichtung und des Bewegungsbetrags des chirurgischen Teilabschnitts (21b), die auf Basis der von den Schätzungsteilabschnitten geschätzten Position ermittelt werden, so antreibt, dass das chirurgische Instrument (21) in die Position zurückgebracht wird, die der chirurgische Teilabschnitt (21b) annimmt, bevor das Endoskop (1) bewegt wird.

5. Vorrichtung zum Steuern der Position eines chirurgischen Instruments für das Endoskop nach Anspruch 1, ferner umfassend:

einen Magnetfelderzeugungsteilabschnitt (61), der im Einführteilabschnitt (3a) des Endoskops (1) bereitgestellt ist;

wobei der zweite Schätzungsteilabschnitt so ausgelegt ist, dass er ein vom Magnetfelderzeugungsteilabschnitt (61) erzeugtes Magnetfeld erkennt, eine Position des chirurgischen Teilabschnitts (21b) anhand einer Position des Magnetfelderzeugungsteilabschnitts (61) auf Basis des erkannten Magnetfelds ermittelt und eine Bewegungsrichtung und einen Bewegungsbetrag des chirurgischen Teilabschnitts (21b) in Verbindung mit einer Bewegung des Endoskops (1) schätzt.

**6.** Vorrichtung zum Steuern der Position eines chirurgischen Instruments für das Endoskop nach Anspruch 1, die ferner so konfiguriert ist, dass die Schätzungsteilabschnitte des chirurgischen Instruments (21), wenn geschätzt wird, dass die Bewegung des Endoskops (1) parallel zu einer Bildaufnahmefläche eines Bildaufnahmeteilabschnitts (7) verläuft, der an einer distalen Endseite (3e) des Einführteilabschnitts (3a) bereitgestellt ist, eine Distanz in einer Tiefenrichtung orthogonal zur Bildaufnahmefläche auf eine Brennrichtung eines optischen Systems im Bildaufnahmeteilabschnitt fixieren, um die Bewegungsrichtung und den Bewegungsbetrag des chirurgischen Teilabschnitts (21b) zu schätzen.

**7.** Vorrichtung zum Steuern der Position eines chirurgischen Instruments für das Endoskop nach Anspruch 1, wobei das chirurgische Instrument (21) durch den Einführteilabschnitt (3a) des Endoskops (1) einführbar ist, um vorwärts und rückwärts beweglich zu sein, wobei der Einführteilabschnitt (3a) einen elektrisch betriebenen Antriebsmechanismus (71) aufweist, der so ausgelegt ist, dass er einen Biegeteilabschnitt (3b), der an einem distalen Ende des Einführteilabschnitts (3a) des Endoskops (1) bereitgestellt ist, in eine gewünschte Richtung biegt.

**8.** Vorrichtung zum Steuern der Position eines chirurgischen Instruments für das Endoskop nach Anspruch 1, wobei die Schätzungsteilabschnitte so ausgelegt sind, dass sie eine Position des chirurgischen Instruments (21) halten, wenn der Bewegungsbetrag des Einführteilabschnitts (3a) des Endoskops (1) kleiner als ein vorab eingestellter Schwellenwert ist.

**9.** Vorrichtung zum Steuern der Position eines chirurgischen Instruments für das Endoskop nach Anspruch 1, **dadurch gekennzeichnet, dass** der Steuerteilabschnitt (22) so ausgelegt ist, dass er für eine optional spezifizierte Zielstelle auf einem Bildschirm auf Bildern, die von einem Bildaufnahmeteilabschnitt (7) aufgenommen werden, eine Bewegung des distalen Endes (3e) des Endoskops (1) zwischen zeitlich sequentiellen benachbarten Bildern schätzt, um eine Größenordnung eines Translationsvektors in der Bewegung zu ermitteln, um eine absolute Bewegung des distalen Endes (3e) des Endoskops (1) zu berechnen.

**10.** Vorrichtung zum Steuern der Position eines chirurgischen Instruments für das Endoskop nach Anspruch 1, wobei der Einführteilabschnitt (3a) des Endoskops (1) einen elektrisch betriebenen Antriebsmechanismus aufweist, der eine Gelenkstruktur mit einer Mehrzahl von Gelenken umfasst, wobei jedes davon durch Ziehen an einem Kabel, das mit einer an einer Welle eines Motors angebrachten Riemenscheibe verbunden ist, und Lockern dieses gebogen wird, wobei der elektrisch betriebene Antriebsmechanismus so ausgelegt ist, dass er vom Motor gemäß einer Anweisung vom Steuerteilabschnitt (42c) angetrieben wird, um einen Bewegungsvorgang und einen Stellungshaltevorgang durchzuführen.

**11.** Vorrichtung zum Steuern der Position eines chirurgischen Instruments für das Endoskop nach Anspruch 4, **dadurch gekennzeichnet, dass** ein elektrisch betriebener Antriebsmechanismus (71) für den Einführteilabschnitt (3a) des Endoskops (1) und der elektrisch betriebene Antriebsmechanismus für den beweglichen Teilabschnitt (21c) des chirurgischen Teilabschnitts (21b) Einrichtungen vom Master-Slave-Typ sind, die jeweils eine Eingabeeinrichtung (25, 78) umfassen, ausgelegt, um von einem Chirurgen manipuliert zu werden, um Positionen und Stellungen des Einführteilabschnitts und des chirurgischen Teilabschnitts (21b) fernzuspezifizieren.

**12.** Vorrichtung zum Steuern der Position eines chirurgischen Instruments für das Endoskop nach Anspruch 4, wobei die Schätzungsteilabschnitte so konfiguriert sind, dass sie den Bewegungsbetrag des chirurgischen Teilabschnitts (21b) auf Basis von Betriebsbeträgen von Biege-, Dreh- und Vorwärts- und Rückwärtsbewegungen jedes der Gelenke schätzen.

**Revendications**

**1.** Appareil de commande de position d'instrument chirurgical (2) pour un endoscope (1) comprenant :

un corps d'endoscope (3) permettant à un tissu biomédical dans une cavité corporelle d'être observé ;

un instrument chirurgical (21) ayant une section chirurgicale (21b) qui est mobile d'un seul tenant avec un mouvement d'une extrémité distale (3e) de l'endoscope (1), et qui est mobile vers l'avant et vers l'arrière à travers un canal d'une section d'insertion (3a) de l'endoscope (1) et qui permet au tissu biomédical d'être opéré, et une section mobile (21c) pour courber et déplacer vers l'avant et vers l'arrière la section chirurgicale (21b) ;

un mécanisme d'entraînement (23) adapté pour courber et déplacer vers l'avant et vers l'arrière la section mobile (21c) de la section chirurgicale (21b) pour déplacer une position de la section chirurgicale (21b) ;

une première section d'estimation (42a) adaptée pour exécuter un traitement arithmétique pour estimer le mouvement de l'extrémité distale (3e) de l'endoscope (1) sur la base de données d'image obtenues par la section de capture d'image (7) de l'endoscope (1) ;

une seconde section d'estimation (42b) adaptée pour estimer une direction de mouvement et une amplitude de mouvement de la section chirurgicale (21b) sur la base du mouvement de l'extrémité distale (3e) de l'endoscope (1) estimé par la première section d'estimation (42a) ; et

une section de commande (42c) adaptée pour commander, par entraînement, le mécanisme d'entraînement (23) sur la base de la direction de mouvement et de l'amplitude de mouvement de la section chirurgicale (21b), ce qui se base sur la direction de mouvement et l'amplitude de mouvement de la section chirurgicale (21b) estimées par la seconde section d'estimation (42b), de telle sorte que la section chirurgicale (21b) est ramenée à une position adoptée par la section chirurgicale (21b) avant le déplacement de l'endoscope (1).

2.  Appareil de commande de position d'instrument chirurgical pour l'endoscope selon la revendication 1, dans lequel la section mobile (21c) de la section chirurgicale (21b) a une structure articulée comprenant une pluralité d'articulations et de tiges reliant les articulations ensemble.

3.  Appareil de commande de position d'instrument chirurgical pour l'endoscope selon la revendication 1, dans lequel :

    la première section d'estimation (42a) est adaptée pour estimer le mouvement du corps d'endoscope (3) ; et
    la seconde section d'estimation (42b) est adaptée pour estimer la direction de mouvement et l'amplitude de mouvement de la section chirurgicale (21b) de l'instrument chirurgical (21) par rapport au mouvement de l'endoscope (1), sur la base d'une relation de position prédéfinie entre le corps d'endoscope (3) et l'instrument chirurgical (21),
    une position de la section chirurgicale (21b) étant estimée sur la base du mouvement de la section chirurgicale (21b) sur une image capturée par une section de capture d'image (7) prévue à une extrémité distale (3e) de la section d'insertion (3a) de l'endoscope (1), d'informations d'angle relatives aux articulations de la section mobile (21c) de l'instrument chirurgical (21), lesquelles informations étant chargées dans la section de commande (42c), et d'un état de l'instrument chirurgical (21) basé sur des informations de rotation estimées et des informations de mouvement de translation estimées.

4.  Appareil de commande de position d'instrument chirurgical pour l'endoscope selon la revendication 1, dans lequel le mécanisme d'entraînement (23) comprend :

    une pluralité de fils (29), une extrémité de chacun desquels étant reliée à une articulation correspondante parmi les articulations de la section mobile (21c) ; et
    un mécanisme d'entraînement à actionnement électrique adapté pour être commandé par la section de commande (42c) et relié à une autre extrémité de chacun des fils (29) pour tirer et relâcher l'un quelconque des fils (29) afin de courber la section mobile (21c) dans une direction souhaitée,
    la section de commande (42c) étant adaptée, sur la base de la direction de mouvement et de l'amplitude de mouvement de la section chirurgicale (21b) déterminées sur la base de la position estimée par les sections d'estimation, pour entraîner le mécanisme d'entraînement à actionnement électrique pour ramener l'instrument chirurgical (21) à la position adoptée par la section chirurgicale (21b) avant le déplacement de l'endoscope (1).

5.  Appareil de commande de position d'instrument chirurgical pour un endoscope selon la revendication 1, comprenant en outre :

    une section de génération de champ magnétique (61) prévue dans la section d'insertion (3a) de l'endoscope (1) ;
    la seconde section d'estimation étant adaptée pour détecter un champ magnétique généré par la section de génération de champ magnétique (61), déterminer une position de la section chirurgicale (21b) à partir d'une position de la section de génération de champ magnétique (61) basée sur le champ magnétique détecté, et estimer une direction de mouvement et une amplitude de mouvement de la section chirurgicale (21b) conjointement avec un mouvement de l'endoscope (1).

**6.** Appareil de commande de position d'instrument chirurgical pour l'endoscope selon la revendication 1, configuré en outre de telle sorte que, lorsqu'il est estimé que le mouvement de l'endoscope (1) est parallèle à une surface de capture d'image d'une section de capture d'image (7) prévue à un côté extrémité distale (3e) de la section d'insertion (3a), les sections d'estimation de l'instrument chirurgical (21) définissent une distance dans une direction de profondeur orthogonale à la surface de capture d'image, par rapport à une direction focale d'un système optique dans la section de capture d'image, pour estimer la direction de mouvement et l'amplitude de mouvement de la section chirurgicale (21b).

**7.** Appareil de commande de position d'instrument chirurgical pour l'endoscope selon la revendication 1, dans lequel l'instrument chirurgical (21) est apte à être inséré à travers la section d'insertion (3a) de l'endoscope (1) de façon à être mobile vers l'avant et vers l'arrière, la section d'insertion (3a) ayant un mécanisme d'entraînement à actionnement électrique (71) adapté pour courber, dans une direction souhaitée, une section de courbure (3b) prévue à une extrémité distale de la section d'insertion (3a) de l'endoscope (1).

**8.** Appareil de commande de position d'instrument chirurgical pour l'endoscope selon la revendication 1, dans lequel les sections d'estimation sont adaptées pour maintenir une position de l'instrument chirurgical (21) si l'amplitude de mouvement de la section d'insertion (3a) de l'endoscope (1) est inférieure à un seuil prédéfini.

**9.** Appareil de commande de position d'instrument chirurgical pour l'endoscope selon la revendication 1, **caractérisé par le fait que**, pour un site cible éventuellement spécifié dans un écran dans des images capturées par une section de capture d'image (7), la section de commande (22) est adaptée pour estimer un mouvement de l'extrémité distale (3e) de l'endoscope (1) entre des images adjacentes en séquence temporelle pour déterminer une amplitude d'un vecteur de translation dans le mouvement pour calculer un mouvement absolu de l'extrémité distale (3e) de l'endoscope (1).

**10.** Appareil de commande de position d'instrument chirurgical pour l'endoscope selon la revendication 1, dans lequel la section d'insertion (3a) de l'endoscope (1) a un mécanisme d'entraînement à actionnement électrique comprenant une structure articulée ayant une pluralité d'articulations dont chacune est courbée en tirant et relâchant un fil couplé à une poulie fixée à un arbre d'un moteur, le mécanisme d'entraînement à actionnement électrique étant adapté pour être entraîné par le moteur selon une instruction provenant de la section de commande (42c) pour réaliser une opération de mouvement et une opération de maintien de posture.

**11.** Appareil de commande de position d'instrument chirurgical pour l'endoscope selon la revendication 4, **caractérisé par le fait qu'**un mécanisme d'entraînement à actionnement électrique (71) pour la section d'insertion (3a) de l'endoscope (1) et le mécanisme d'entraînement à actionnement électrique pour la section mobile (21c) de la section chirurgicale (21b) sont des dispositifs de type maître/esclave comprenant chacun un dispositif d'entrée (25, 78) adapté pour être manipulé par un chirurgien pour spécifier à distance des positions et des postures de la section d'insertion et de la section chirurgicale (21b).

**12.** Appareil de commande de position d'instrument chirurgical pour l'endoscope selon la revendication 4, dans lequel les sections d'estimation sont configurées pour estimer l'amplitude de mouvement de la section chirurgicale (21b) sur la base d'amplitudes d'actionnement de courbure, de rotation et de mouvements vers l'avant et vers l'arrière de chacune des articulations.

EP 2 130 479 B1

FIG.1

```
            ┌─────────────┐
            │    Start    │
            └─────────────┘
                   │
                   ▼
      ┌───────────────────────────┐
      │   Estimate motion of distal │ ─── S1
      │      end of endoscope       │
      │(estimate translation and rotation)│
      └───────────────────────────┘
                   │
                   ▼
      ┌───────────────────────────┐
      │  Estimate motion of surgical│ ─── S2
      │    section based on motion  │
      │   of distal end of endoscope│
      └───────────────────────────┘
                   │
                   ▼
      ┌───────────────────────────┐
      │ Calculate control amount for each│ ─── S3
      │  joint required to move surgical │
      │   section to original position   │
      └───────────────────────────┘
                   │
                   ▼
      ┌───────────────────────────┐
      │    Move surgical section   │ ─── S4
      └───────────────────────────┘
                   │
                   ▼                    S5
                ◇─────────────────────◇
        YES ───  Process to be continued?
                ◇─────────────────────◇
                   │
                   │ NO
                   ▼
            ┌─────────────┐
            │     End     │
            └─────────────┘
```

F I G. 2

3a

7

12

21c

21b

(x″ y″ z″)

(x y z)
(x′ y′ z′)

F I G. 3A

F I G. 3B

(x y z)

F I G. 3C

(x' y' z')          (x" y" z")

**FIG. 4**

First estimation section
Second estimation section
Control section
CPU
Memory

Outside body

Body cavity

P(xP  yP  f)

P'(x'P  yP  f)

q'(x'q  yq  f)

Q'(x'Q  yQ z'Q)

F I G. 5A

F I G. 5B

F I G. 5C

First estimation section

Second estimation section

Control section

CPU

Memory

Bending control section

Driver

W

Outside body

Body cavity

EP 2 130 479 B1

F I G. 6

Start

Estimate motion V of distal
end of endoscope — S11

$|V| < V_{thr}$ — S12

YES

NO

Estimate motion of surgical
section based on motion of distal
end of endoscope — S13

Calculate motion of joints, parallel
movement, and rotational amount
required to move surgical
section back to original position — S14

Move surgical section — S15

Process to be continued? — S16

YES

NO

End

F I G. 7

**EP 2 130 479 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3007715 B
- JP 2003127076 A
- WO 2007005367 A2
- US 2004138525 A
- US 2005228221 A
- US 5432543 A
- JP 3347385 B
- JP 9026547 A
- JP 3571675 B